# EUROPÄISCHE PATENTSCHRIFT

(11) **EP 3 510 408 B1**
(45) Veröffentlichungstag und Bekanntmachung des Hinweises auf die Patenterteilung: **14.10.2020**
(21) Anmeldenummer: 17767985.9
(22) Anmeldetag: 21.08.2017
(51) Int. Cl.: G01N 33/68, G01N 33/12

(54) **VERFAHREN ZUM QUALITATIVEN NACHWEIS VON ZUGESETZTEN EIWEISS-STOFFEN IN LEBENSMITTELN**
METHOD FOR QUALITATIVE DETECTION OF ADDED PROTEINS IN FOODS
PROCÉDÉ POUR LA DÉTECTION QUALITATIVE DE PROTÉINES AJOUTÉES DANS DES DENRÉES ALIMENTAIRES

(30) Priorität: 12.09.2016 DE 102016010887
(43) Veröffentlichungstag der Anmeldung: 17.07.2019
(73) Patentinhaber: Membrapure Gesellschaft Für Membrantechnik mbH, 16761 Hennigsdorf (DE)
(72) Erfinder: FLINDT, Erdmann, 14193 Berlin (DE)
(74) Vertreter: Habenicht, Wieland
(86) Internationale Anmeldenummer: PCT/DE2017/000256
(87) Internationale Veröffentlichungsnummer: WO 2018/046033

(56) Entgegenhaltungen:
- WO-A1-96/35125
- WO-A1-2008/003388

## Beschreibung

Die Erfindung betrifft ein Verfahren zum qualitativen Nachweis von zugesetzten Eiweiß-Stoffen in Lebensmitteln, nämlich von Blutplasma in Fleisch und Fleischwaren, wobei die Lebensmittel bei der Probenaufbereitung in Präzipitat- und Fluid-Phase getrennt werden.

Die Lebensmittelchemie ist ein Zweig der angewandten Chemie, der sich insbesondere mit der Ermittlung der Zusammensetzung, der Funktion von Lebensmitteln und deren einzelnen Komponenten befasst. Die Überwachung der Hersteller, des Handels und des Verkehrs mit Lebensmitteln und der Nachweis möglicher Täuschung und Irreführung des Verbrauchers sind Schwerpunkte dieses Gebietes. Qualitative und quantitative Analysen von Lebensmitteln auf erlaubte oder verbotene Lebensmittelzusatzstoffe, auf Rückstände von Pestiziden, Düngemitteln, Lösungsmitteln, Antibiotika, Hormonen und Schwermetallen sind seit vielen Jahren bekannte Untersuchungsparameter.

Durch die Globalisierung des zwischenstaatlichen Handels, von dem auch die Lebensmittelindustrie in starkem Maße betroffen ist, sind zuverlässige und reproduzierbare Analysemethoden unerlässlich. Analytikbedarf besteht für die gesamte Kette vom Hersteller, der seine Rohwaren überprüfen möchte, über den Handel, der möglichst viele und verlässliche Qualitätsparameter beim Einkauf seiner Produkte wünscht, bis zum Konsumenten, der qualitativ einwandfreie und authentische Produkte erwerben möchte. Eiweißhaltige Zusätze in Form von hochmolekularen Fleischpulvern bzw. Eiweißhydrolysaten gibt es seit über 30 Jahren und kommen ursprünglich aus dem asiatischen Raum. Sie dürfen nach der Fleischverordnung in Fleischereierzeugnissen nicht verwendet werden. Ausgenommen sind pflanzliche Eiweiße, Gelatine für genau definierte Fleischerzeugnisse und Gewürze, die ihrer Definition hinsichtlich Gesamtstickstoffgehalt und Anteile von Aminosäurestickstoff entsprechen müssen. Bei Verwendung solcher Zusätze ist auf jeden Fall eine Deklaration im Zutatenverzeichnis zwingend vorgeschrieben. Der Einsatz von Blutplasma ist ebenfalls nur für wenige und genau definierte Fleischwaren zulässig, wobei die Verwendungseinschränkung in den Leitsätzen der Fleischverordnung genau geregelt ist. Auch in zulässigen Produkten muss die Verwendung von Blutplasma im Zutatenverzeichnis deklariert werden. Von unzulässigen Zusätzen von Eiweißhydrolysaten und Blutplasma sowie von Deklarationsverstößen sind viele Produkte aus dem Fleisch- und Wurstwarenbereich betroffen. Durch den Einsatz von Eiweißhydrolysaten lässt sich zusätzlich Wasser ins Produkt einführen, ohne dass sich das Eiweiß-Wasser-Verhältnis völlig verschiebt. Insbesondere bei Wurstwaren kann die Stickstoffbilanz dadurch nach oben verschoben werden, so dass sich der Anteil an wertbestimmendem Muskelfleisch reduzieren lässt.

Blutplasma, welches naturbedingt ein hohes Wasserbindungsvermögen aufweist, ermöglicht ebenfalls außergewöhnliche Wassereinträge in verschiedenste Produkte. So lassen sich beispielsweise die Produktionsverluste durch Garung beim Kochschinken und anderer Kochpökelwaren reduzieren bzw. sogar überkompensieren, das heißt, dass die Endprodukte schwerer als die Ausgangsprodukte vor der Garung sind.

In Journal of Chromatography (1993) Volumen 615, Seiten 1 bis 22, G. Sarwar, H. G. Botting, werden verschiedene Verfahren zur Bestimmung des Gehalts von Aminosäuren in Fleischproben mittels eines hydrolytischen Aufschlusses beschrieben.

In "Lehrbuch der Lebensmittelchemie", 5. Auflage, Springer-Verlag, Berlin, (2001), Seiten 601 bis 602, H.-D. Belitz, W. Grosch, P. Schieberle, sowie in WO 83/03673 A1, WO 87/07722 A1 und DE 26 18 052 A1 werden verschiedene Verfahren zur Lebensmittelanalyse beschrieben, bei denen der Aminosäuregehalt der in einer Lebensmittelprobe enthaltenen Proteine nach Hydrolyse ermittelt wird.

Nguyen Q et al "Comparision of the Amino-ACID Composition of two commercial porcine skins (rind)", Journal of Agriculture and food chemistry, American chemical Society, US, Bd. 34, Nr. 3, 1. Januar 1986 (1986-01-01), Seiten 565-572, XP002468288, ISSN: 0021-8561, DOI: 10.1021/ JF00069A048, offenbart die Analyse von Hydroxylysin in nicht-tierischen und tierischen Eiweißhydrolysaten nach Säurehydrolyse und lehrt, das Hydroxylysin in den nicht-tierischen Eiweißzusätzen nicht nachweisbar ist, während es ein Marker für Zusätze tierischen Ursprungs ist.

Aus Littmann-Nienstedt ist "Nachweis und Bestimmung von hydrolysierter Gelatine in Fleischerzeugnissen und Zusatzmitteln für Fleicherzeugnisse" in Deutsche Lebensmittel-Rundschau 2001, 97. Jahrgang, Heft 2, Seiten 60 bis 64, offenbart ein Verfahren zum Nachweis des für Fleischerzeugnisse unzulässigen Zusatzes von hydrolysierter Gelatine. Die Probe wird wie bei der Bestimmung des Nicht-Proteinstickstoffgehaltes mit Trichloressigsäure extrahiert, wobei in diesem Extraktionsmittel auch hydrolysierte Gelatine löslich ist, die charakteristisch hohe Mengen an Hydroxyprolin enthält. In einem Teil des Extraktes wird der Gehalt an Hydroxyprolin bestimmt. Nicht löslich in 20 %iger Trichloressigsäure ist das von Natur aus im Fleisch enthaltene Bindegewebe Eiweiß, in dem ebenfalls die Aminosäure Hydroxyprolin in charakteristisch hohen Mengen enthalten ist. Der aus diesem Teil des Fleischerzeugnisses stammende Gehalt an Hydroxyprolin wird also mit dieser Methode nicht erfasst.

WO2008/003388 A1 offenbart ein Verfahren zum qualitativen Nachweis von zugesetzten Eiweiß-Stoffen in Lebensmitteln, insbesondere von eingesetzten Eiweiß-Hydrolysaten oder Blutplasma in Fleisch und Fleischwaren, wobei die Lebensmittel bei der Probenaufbereitung in Präzipitat- und Fluid-Phase getrennt werden, wobei das Verfahren dadurch gekennzeichnet ist, dass ein Teil der Fluid-Phase und / oder zumindest ein Teil der Präzipitat-Phase hydrolysiert wird und die auf diese Weise erhaltenen freigesetzten Aminosäuren qualitativ und quantitativ bestimmt werden, wobei die ermittelten Probengehalte der Fluid-Phasen und / oder Präzipitat-Phasen freigesetzten Aminosäuren miteinander verglichen werden mit solchen Probengehalten, die aus den entsprechenden, nicht mit Eiweiß-Hydrolysaten und / oder Blutplasma behandelten Lebensmitteln stammen.

WO96/35125 A1 offenbart ein Verfahren zum qualitativen Nachweis von Eiweiß-Hydrolysaten in Lebensmitteln, insbesondere von zugesetzten tierischen und pflanzlichen Eiweiß-Hydrolysaten in Fleisch und Fleischwaren, wobei die Lebensmittel zu freien Aminosäuren enthaltenden Proben aufbereitet werden und die in den aufbereiteten Proben vorhandenen freien Aminosäuren qualitativ und quantitativ bestimmt werden, wobei die ermittelten Probengehalte an freien Aminosäuren mit solchen verglichen werden, die aus den entsprechenden nicht mit Eiweiß-Hydrolysaten behandelten Lebensmitteln stammen.

DE 195 16 077 C1 offenbart ein Verfahren zum qualitativen Nachweis von zugesetzten Eiweißhydrolysaten in Lebensmitteln, insbesondere von zugesetzten tierischen und pflanzlichen Eiweißhydrolysaten in Fleisch und Fleischwaren, das dadurch gekennzeichnet ist, dass die Lebensmittel zu freie Aminosäuren enthaltenden Proben aufbereitet werden und die in den aufbereiteten Proben vorhandenen freien Aminosäuren qualitativ und quantitativ bestimmt werden, wobei die ermittelten Probengehalte an freien Aminosäuren mit solchen Probengehalten verglichen werden, die aus den entsprechenden, nicht mit Eiweißhydrolysaten behandelten Lebensmitteln stammen.

Mit Hilfe solcher Verfahren ist es zwar möglich, schon teilweise gezielte Aussagen über Zusätze von Eiweißdydrolysaten in Fleisch und Fleischwaren zu treffen, jedoch sind diese Aussagen in nicht wenigen Fällen unvollständig, so dass nach wie vor ein Bedürfnis an einer weiteren und genaueren Analytik in diesem Bereich besteht.

Aus dem Stand der Technik ist somit bis heute kein hochgradig zuverlässiges und breitbandig angelegtes Analyseverfahren zur Bestimmung von zugesetzten tierischen und pflanzlichen Eiweißhydrolysaten in Lebensmitteln, insbesondere Fleisch und Fleischwaren, bekannt. Insbesondere der Nachweis von Schweineblutplasma ist bislang ebenfalls nicht möglich.

Das der Erfindung zugrundeliegende Problem ergibt sich somit darin, ein Verfahren zum qualitativen Nachweis von zugesetztem Blutplasma in Lebensmitteln bereitzustellen, das eine Vielzahl solcher Zugaben sicher und breitbandig erfasst.

Dieses Problem wird erfindungsgemäß durch ein Verfahren nach Anspruch 1 und eine Verwendung nach Anspruch 14 gelöst.

Beim erfindungsgemäßen Verfahren werden die Lebensmittel, insbesondere Fleisch und Fleischwaren bei der Probenaufbereitung in Präzipitat- und Fluid-Phase getrennt. Dies geschieht beispielsweise und insbesondere durch Zerkleinerung einer Probe und Homogenisierung mit Wasser, wobei das Homogenisat mit Sulfosalicylsäure versetzt wird, so dass hochmolekulare Proteine innerhalb einer bestimmten Zeit bei einer bestimmten Temperatur (beispielsweise 30 Minuten bei +4°C im Kühlschrank) ausfallen und im Anschluss die Probe mit einem Papierfilter filtriert wird, wobei die ausgefallenen Proteine als Filterrückstand als Präzipitat-Phase übrig bleiben, während das Filtrat als solches als Fluid-Phase vorliegt. Die Fluid-Phase enthält freie Aminosäuren, wobei diese qualitativ und quantitativ bestimmt werden, wobei die ermittelten Probengehalte dieser Aminosäuren verglichen werden mit solchen Probengehalten, die aus den entsprechenden, nicht mit Blutplasma behandelten Lebensmitteln stammen. Erfindungsgemäß werden bei der Untersuchung auf zugesetztes Blutplasma in der Fluidphase die Aminosäuren Ornithin und Cystin berücksichtigt.

Weiterhin ist es von Vorteil, die aus zumindest einem Teil der Fluid-Phase stammenden freien Aminosäuren qualitativ und quantitativ zu bestimmen, wobei die ermittelten Probengehalte dieser freien Aminosäuren verglichen werden mit solchen Probengehalten, die aus den entsprechenden, nicht mit Blutplasma behandelten Lebensmitteln stammen, um weitere Informationen über die Lebensmittel zu erhalten.

Aufgrund der teilweise summarischen jedoch vielfältigen Typen- und Konzentrationsänderungen der einzelnen Aminosäuren können über kombinierende Korrelationsanalyse der einzelnen Aminosäuren und deren Konzentrationen entsprechende Rückschlüsse durch Vergleich von unbehandelten mit unbehandelten Referenzproben und bewusst behandelten Proben schließlich entsprechend zumindest qualitative Aussagen gemacht werden, die dann bei einer entsprechenden unbekannten Lebensmittelprobe über die vorher bekannten Korrelationsregeln quasi retro-analytisch Aussagen in qualitativer Hinsicht über Reinheit bzw. Zugabe von Blutplasma zulassen.

Beim erfindungsgemäßen Verfahren werden die zu untersuchenden Lebensmittel auf die oben genannte Art und Weise über die entsprechenden Trennungsschritte zu freie Aminosäuren enthaltenden Proben aufbereitet, die anschließend einer entsprechenden Aminosäurebestimmung unterworfen werden, um die freien Aminosäuren und deren Gehalte zu bestimmen. Die Probenaufbereitung sowie die Aminosäurebestimmung verlaufen nach konventionellen Methoden. Beispielsweise werden die Lebensmittel zunächst genau eingewogen, mit destilliertem Wasser verdünnt, homogenisiert und anschließend die Fluid-Phase mit den darin enthaltenen freien Aminosäuren ionenaustausch-chromatographisch getrennt und nach Farbreaktion mit Ninhydrin oder Ortho-Phthal-dialdehyd nachgewiesen. Dieser Vorgang ist in der Regel automatisiert. Selbstverständlich ist es auch denkbar, dass die Aminosäuren z. Bsp. Phenylsenföl, FMOC, Dabsylchlorid oder Dansylclorid derivatisiert und einer Flüssigkeits-chromatographie, insbesondere einer HPLC (High Performance Liquid Chromatography) mit Reversed-Phase-Characteristic unterworfen werden. Die erhaltenen Chromatogramme werden sowohl qualitativ als auch quantitativ ausgewertet. Die zu den einzelnen Proben ermittelten Mengenangaben der freien Aminosäuren, die aus mit Blutplasma behandelten Lebensmitteln stammen, werden mit solchen Probenmengenangaben verglichen, die aus entsprechenden unbehandelten Lebensmitteln stammen, denen also kein Blutplasma zugesetzt worden sind. Überschreiten einige Aminosäuregehalte die aus den entsprechenden, unbehandelten Lebensmittelproben ermittelten Werte, so ist dies zumindest ein Indiz für eine unzulässige Blutplasma-Zugabe, jedoch noch kein Beweis, da die in Spezies vorkommenden Aminosäuren immer einer gewissen Schwankungsbreite unterliegen. Bei Vergleich der ermittelten Gehalte der einzelnen Aminosäuren stellte man jedoch überraschenderweise fest, dass einzelne Aminosäuregehalte trotz Zugabe von Blutplasma in Lebensmitteln, insbesondere von Fleisch und Fleischwaren, annähernd konstant bleiben, während andere Aminosäuregehalte sich signifikant verändern, hier Ornithin und Cystin. Diese überraschende Erkenntnis erlaubt somit zumindest den direkten Vergleich der in einer natürlichen Schwankungsbreite vorkommenden, jedoch bei Blutplasmazusatz sich signifikant verändernde Aminosäuregehalte mit aus Erfahrung festgelegten Werten, um Rückschlüsse auf eventuelle Zusätze in Form von Blutplasma zu ziehen bzw. erlaubt, falls ohne Blutplasma versetzte entsprechende Lebensmittel zur Verfügung stehen, sogar den Vergleich der ermittelten Probengehalte an bei Blutplasmazugabe sich mengenmäßig signifikant verändernden Aminosäuren mit Probengehalten an diesen Aminosäuren, die aus nicht mit Blutplasma behandelten Lebensmitteln stammen. Durch die Signifikanz der zur Beurteilung herangezogenen Wertung ist eine sichere Aussage über Blutplasmazusatz möglich.

Dadurch, dass bei Blutplasmazusatz (selbstredend immer tierischen Ursprungs) bestimmte Aminosäuregehalte sich signifikant verändern, während andere nahezu unverändert bleiben, ist es zweckmäßig, für einen Zusatz von Blutplasma Probengehalte von freien Aminosäuren, die sich durch den Zusatz erhöhen, mit Probengehalten freier Aminosäuren, die durch den Zusatz praktisch unverändert bleiben, zur Durchführung des Vergleichs in Beziehung zu setzen. Erfindungsgemäß konnte gefunden werden, dass sich Ornithin und Cystin verändern. Der Quotient der Ornithin- und Cystin-Menge bleibt bei Zusatz von Blutplasma nahezu konstant und stellt einen weiteren erfindungsgemäß wichtigen Indikator dar. Die Quotientenbildung ermöglicht es, Proben auffällig als mit (tierischem) Blutplasma behandelt zu identifizieren, deren Aminosäuregehalte trotz unzulässiger Zusätze noch in der natürlichen Schwankungsbreite liegen und somit auch diese Grenzfälle sicher erfasst werden.

Erfindungsgemäß handelt es sich beim Verfahren um ein, mehrere Eluenten und ein Trennprogramm verwendendes chromatographisches Verfahren, da auf diese Weise eine sehr dezidierte Ausdifferenzierung hinsichtlich der Aminosäuren möglich ist.

In diesem Kontext ist es erfindungsgemäß, wenn es sich bei den Eluenten um folgende handelt, mit jeweils folgender Zusammensetzung:
A: Lithiumacetat-Dihydrat 6,65 g, Ethanol 10 ml, Trifluoressigsäure-Lsg 50 % 8 ml, Caprylsäure 0,1 ml, Reinstwasser 470 ml.
B: Lithiumhydroxid-Monohydrat 2,5 g, Borsäure 0,5 g , Ethanol 5 ml, Caprylsäure 0,1 ml, Trifluoressigsäure-Lsg.50% 4,7 ml, Reinstwasser 480 ml.
C: Lithiumhydroxid-Monohydrat 2,1 g, Borsäure 1,5 g Ameisensäure 1,5 ml, Essigsäure 100% 1,5 ml, Caprylsäure 0,1 ml, Trifluoressigsäure-Lsg. 50% 0,8 ml, Reinstwasser 495 ml.
D: Di-Lithiumtetraborat 2,0 g, Borsäure 2,5 g, Ethylendiamin-Tetraessigsäure Di-Lithiumsalz Hydrat 0,13 g, Lithiumhydroxid-Monohydrat 3,18 g, Caprylsäure 0,1 ml, Trifluoressigsäure 50% 3 ml, Reinstwasser 483 ml.
E: Di-Lithiumtetraborat 1,0 g, Borsäure 1,5 g, LithiumhydroxidMonohydrat 5,25 g, Caprylsäure 0,1 ml, Trifluoressigsäure 50% 3,5 ml, Reinstwasser 490 ml,
wobei die Angaben hinsichtlich der Zusammensetzung bezüglich der Bestandteile relativ zueinander zu verstehen sind. Beispielsweise können die Mengenangaben der einzelnen Bestandteile eines Eluenten verdoppelt werden, da sich in der relativen Zusammensetzung der Bestandteile zueinander nichts ändert.

Weiterhin ist es in diesem Kontext vorteilhaft, da bewährt, wenn die Eluenten bei den nachfolgenden pH-Werten verwendet werden: Eluent A pH 2,96, Eluent B pH 3,46, Eluent C pH 4,48, Eluent D pH 7,76, Eluent E pH 10,42.

Darüber hinaus hat es sich in der Praxis bewährt, wenn die Eluenten A bis E nacheinander in der Reihenfolge A, B, C, D, E verwendet werden.

Von besonderem Vorteil, da in der Praxis bewährt, ist es, wenn das folgende Trennprogramm verwendet wird, wobei die Daten sich auf eine Eluentenflussrate von 220 µl/min und folgenden Eluenten- und Säulentemperaturverlauf beziehen: Gesamtlaufzeit: 205 Minuten, 20 Sekunden;
Eluentenverlauf:
- Eluent A Zeit ab 0 Stunden, 0 Minuten bis 36 Minuten, 35 Minuten
- Eluent B Zeit ab 36 Minuten, 35 Sekunden bis 1 Stunde, 2 Minuten, 42 Sekunden
- Eluent C Zeit ab 1 Stunde, 2 Minute, 42 Sekunden bis 1 Stunde, 32 Minuten, 21 Sekunden
- Eluent D Zeit ab 1 Stunde, 32 Minuten, 21 Sekunden bis 2 Stunden, 2 Minuten, 9 Sekunden
- Eluent E Zeit ab 2 Stunden, 2 Minuten, 9 Sekunden bis 2 Stunden, 51 Minuten, 36 Sekunden
- Säulentemperaturverlauf:
   - Zeit: 0 Stunden, 0 Minuten bis 12 Minuten, 40 Sekunden; Säulentemperatur: + 33°C bei 0 Stunden, 0 Minuten; + 33°C bei 12 Minuten, 40 Sekunden;
   - Zeit: 12 Minuten, 40 Sekunden bis 36 Minuten, 36 Sekunden; Säulentemperatur: + 33°C bei 12 Minuten, 40 Sekunden; + 33°C bei 36 Minuten, 36 Sekunden;
   - Zeit: 36 Minuten, 36 Sekunden bis 47 Minuten, 02 Sekunden; Säulentemperatur: + 33°C bei 36 Minuten, 36 Sekunden; + 44°C bei 47 Minuten, 02 Sekunden;
   - Zeit: 47 Minuten, 02 Sekunden bis 1 Stunden, 11 Minuten, 15 Sekunden; Säulentemperatur: + 44°C bei 47 Minuten, 02 Sekunden; + 33°C bei 1 Stunde, 11 Minuten, 15 Sekunden;
   - Zeit: 1 Stunde, 11 Minuten, 15 Sekunden bis 1 Stunde, 43 Minuten, 57 Sekunden; Säulentemperatur: + 33°C bei 1 Stunde, 11 Minuten, 15 Sekunden; + 59°C bei 1 Stunde, 43 Minuten, 57 Sekunden;
   - Zeit: 1 Stunde, 43 Minuten, 57 Sekunden bis 2 Stunden, 06 Minuten, 34 Sekunden; Säulentemperatur: + 59°C bei 1 Stunde, 43 Minuten, 57 Sekunden; + 40°C bei 2 Stunden, 06 Minuten, 34 Sekunden;
   - Zeit: 2 Stunden, 06 Minuten, 34 Sekunden bis 2 Stunden, 58 Minuten, 15 Sekunden; Säulentemperatur: + 40°C bei 2 Stunden, 58 Minuten, 15 Sekunden; + 66°C bei 2 Stunden, 58 Minuten, 15 Sekunden;
   - Zeit: 2 Stunden, 58 Minuten, 15 Sekunden bis 3 Stunden, 04 Minuten, 56 Sekunden; Säulentemperatur: + 66°C bei 2 Stunden, 58 Minuten, 15 Sekunden; + 70°C bei 3 Stunden, 04 Minuten, 56 Sekunden;
   - Zeit: 3 Stunden, 04 Minuten, 56 Sekunden bis 3 Stunden, 13 Minuten, 36 Sekunden; Säulentemperatur: + 70°C bei 3 Stunden, 04 Minuten, 56 Sekunden; + 70°C bei 3 Stunden, 13 Minuten, 36 Sekunden;
   - Zeit: 3 Stunden, 13 Minuten, 36 Sekunden bis 3 Stunden, 21 Minuten, 22 Sekunden; Säulentemperatur: + 70°C bei 3 Stunden, 13 Minuten, 36 Sekunden; + 35°C bei 3 Stunden, 21 Minuten, 22 Sekunden.

Das erfindungsgemäße Nachweisverfahren ist wirtschaftlich gesehen interessant, da keine zusätzlichen speziell für das Verfahren entwickelten Apparaturen notwendig sind, so dass die sich in einem Laborpark befindenden Geräte verwendet werden können.

Erfindungsgemäß wird weiterhin beansprucht die Verwendung von Ornithin und Cystin im erfindungsgemässen Verfahren als Indikator zum qualitativen Nachweis von zugesetztem Blutplasma in Lebensmitteln in Fleisch und Fleischwaren.

In diesem Kontext ist es vorteilhaft, da in der Praxis bewährt, wenn auch der nahezu konstante Quotient der Molmengen von Ornithin zu Cystin oder umgekehrt berücksichtigt wird.

Vorteilhafterweise ist es von Vorteil, wenn es sich bei den zu untersuchenden Lebensmitteln um Schweinefleisch und/oder um Schweinefleischwaren handelt.

In der Regel liegt der Betriebsdruck der Trennsäule bei 60 - 80 bar.

Das in den Figuren und in den Tabellen gezeigte Beispiel soll die Erfindung in nicht beschränkender Hinsicht näher erläutern.

### Material und Methoden

Material: 20 Proben unterschiedliches Schweinefleisch pur (Gulasch, Kotelett, Schnitzel), Schweinefleisch mit Zusatz 5 % Blutplasma, Schweinefleisch mit Zusatz 10 % Blutplasma.
20 Proben Brühwurst pur, Brühwurst mit Zusatz 5 % Blutplasma, Brühwurst mit Zusatz 10 % Blutplasma. Das zugesetzte Blutplasma hatte eine Konzentration von freien Cystin 108 nmol / ml Plasma und von freien Ornithin 194 nmol/ml Plasma.

Probenaufbereitung: Die Proben wurden mit einen Pürierstab zerkleinert. Für den Nachweis der freien Aminosäuren wurden 10 g der homogenisierten Probe in ein 50 ml Zentrifugenröhrchen eingewogen, mit 10 g Reinstwasser mit Aminosäure Norleucin (externer Standard) versetzt und mit einem Ultra Turrax homogenisiert. Um die Proteine auszufällen, wurden dem Homogenisat 5 g 10%ige Sulfosalicylsäure zugegeben und 30 Minuten bei 4 °C in einen Kühlschrank gestellt. Anschließend wurde die Probe 10 Minuten bei 6000 U/min zentrifugiert. Der Proben-Überstand wurde über einen Papierfaltenfilter 619EH¼ der Firma Macherey-Nagel gereinigt. Zur weiteren Aufreinigung wurde 1 ml des gewonnenen Filtrats für 3 Minuten bei 14.000 U/min unter Verwendung von Zentrifugenfilter (MembraSpin) filtriert. Die Proben wurden bis zur Untersuchung in 1,5 ml Probenröhrchen tiefgefroren gelagert. Zur Analyse der freien Aminosäuren wurden die eingefrorenen Probenlösungen bei Zimmertemperatur aufgetaut. Jeder Probenlösung wurden 500 µl entnommen und 1+1 mit Probenverdünnungspuffer verdünnt. Anschließend wurden die Aminosäurekonzentrationen bestimmt.

### Am inosäureanalytik

Die beiden Aminosäuren Ornithin (Orn) und Cystin (Cys)2 sind relevant für eine aussagefähige Beurteilung. Die Trennung der Aminosäuren erfolgt ionenaustauschchromatographisch mit Hilfe eines Aminosäureanalysator.

Für eine optimale Trennung und Reproduzierbarkeit wurden speziell Trennsäule, Elueten und Trennprogramm entwickelt und eingesetzt. Die Trennsäule gepackt mit Polymerharz aus Styrol und Divinylbenzol und einer Vernetzung von 10%. Die Abmessungen der Trennsäule sind 4 mm im Innendurchmesser und 118 mm Innenlänge und 125 mm Außenlänge

Die 5 Elueten, bezeichnet A, B, C, D, E, zusammengesetzt:
A: aus Lithiumacetat-Dihydrat 6,65 g, Ethanol 10 ml, Trifluoressigsäure-Lsg 50 % 8 ml, Caprylsäure 0,1 ml, Reinstwasser 470 ml.
B: Lithiumhydroxid-Monohydrat 2,5 g, Borsäure 0,5 g, Ethanol 5 ml, Caprylsäure 0,1 ml, Trifluoressigsäure-Lsg. 50% 4,7 ml, Reinstwasser 480 ml.
C: Lithiumhydroxid-Monohydrat 2,1 g, Borsäure 1,5 g, Ameisensäure 1,5 ml, Essigsäure 100% 1,5 ml, Caprylsäure 0,1 ml, Trifluoressigsäure-Lsg. 50% 0,8 ml, Reinstwasser 495 ml.
D: Di-Lithiumtetraborat 2,0 g, Borsäure 2,5 g, EthylendiaminTetraessigsäure Di-Lithiumsalz Hydrat 0,13 g, Lithiumhydroxid-Monohydrat 3,18 g, Caprylsäure 0,1 ml, Trifluoressigsäure 50% 3 ml, Reinstwasser 483 ml.
E: Di-Lithiumtetraborat 1,0 g, Borsäure 1,5 g, LithiumhydroxidMonohydrat 5,25 g, Caprylsäure 0,1 ml, Trifluoressigsäure 50% 3,5 ml, Reinstwasser 490 ml.
mit unterschiedlichen pH-Wert: Eluent A 2,96, Eluent B 3,46, Eluent C 4,48, Eluent D 7,76, Eluent E 10,42.

Als Regenerationslösung (Eluent F) zur Regenerierung der Trennsäule nach Trennung der Aminosäuren aus der Probe, wird eine Lithiumhydroxid-Lösung, pH-Wert 12,43, verwendet.

Die Injektion der Analysenprobe in das System erfolgt automatisch über einen Autosampler und die Einführung der Analysenprobe in die Trennsäule erfolgt mit Eluent A. Die fünf Eluenten und die Regenerationslösung werden nacheinander durch ein 6 Wege-Umschaltventil ausgewählt und auf die Trennsäule mit Hilfe einer Förderpumpe zugeführt.

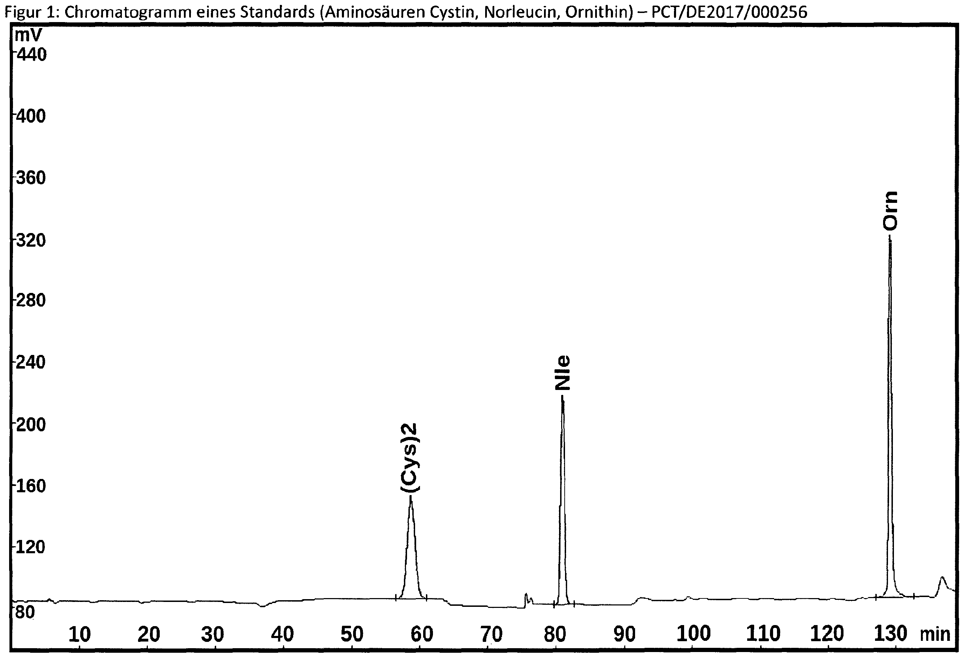

Der pH Wert der Eluenten wird schrittweise von Eluent A nach Eluent F erhöht (siehe Figur 1). Hierbei ist der pH von 2,96 in der ersten Stufe am niedrigsten und erhöht sich bis zur sechsten Stufe Regeneration der Säule mit Eluent F auf 12,46. Die Temperatur wird in mehreren Stufen geändert. Die Regelung der Säulentemperatur (siehe Tabelle 1) ist auf das Verfahren beschränkt und wird über einen Reaktor ausgeführt.

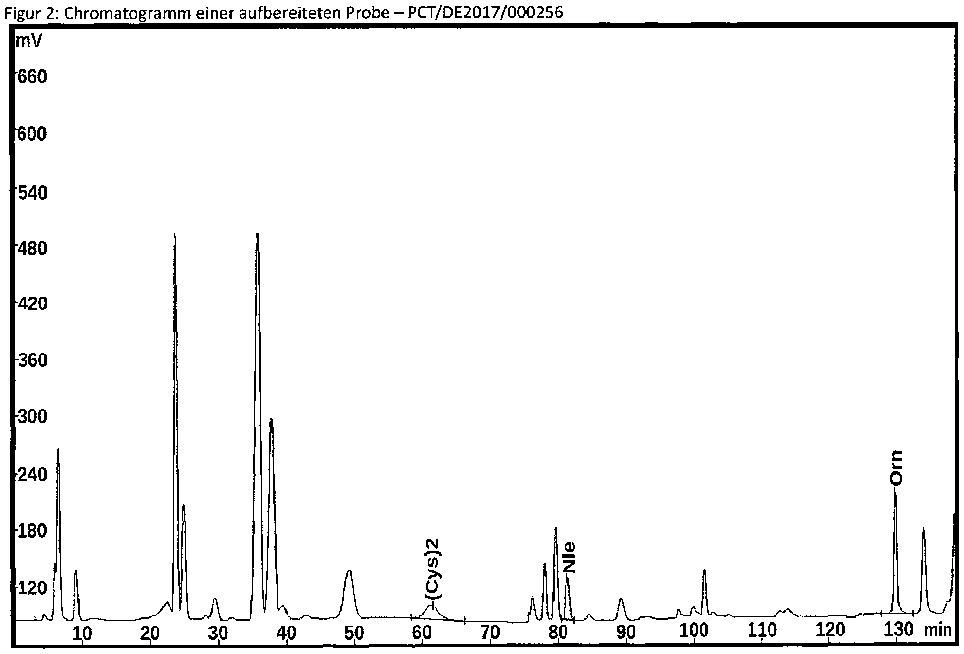

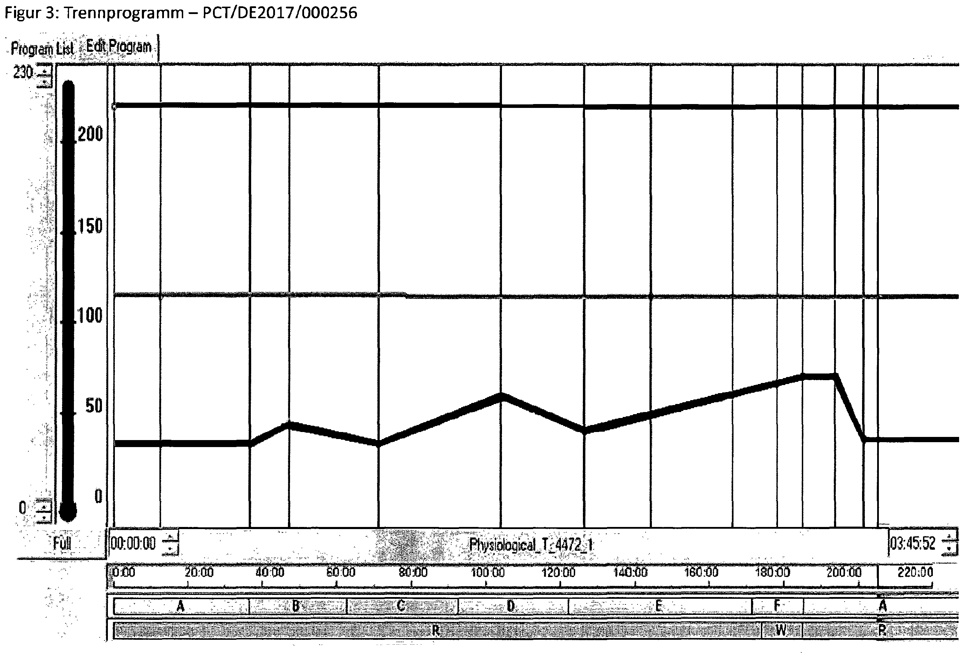

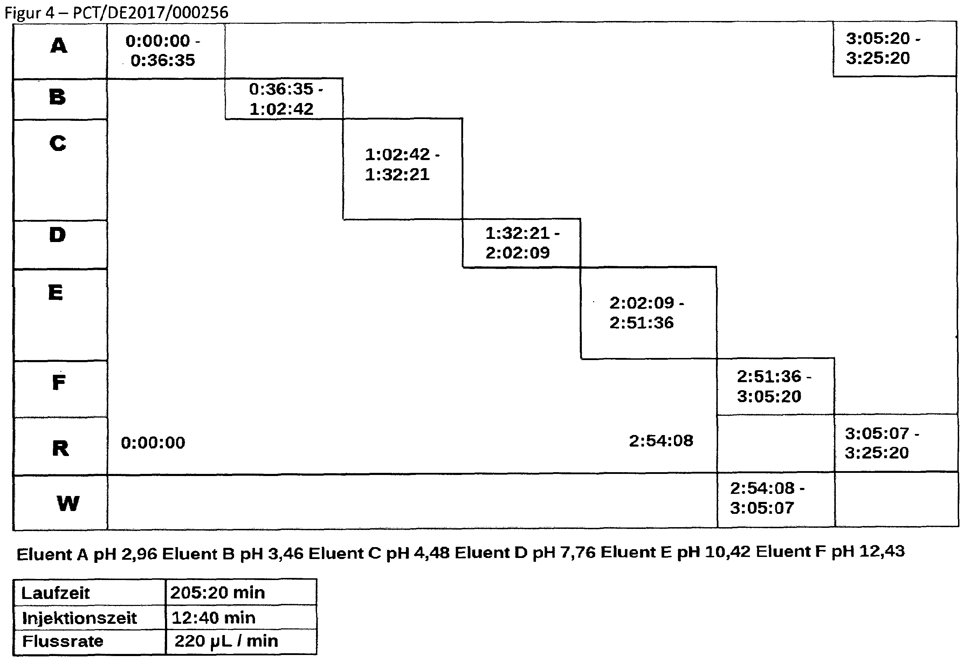

Nach der Trennung reagieren die Aminosäuren mit der Reagenzlösung Ninhydrin zu einem blauen Farbstoff (Ruhemanns Purpur). Die Intensität der Farbe ist der Konzentration des Farbstoffes und damit der Konzentration einer zu bestimmenden Aminosäure proportional. Zur quantitativen Analyse werden die Aminosäuren photometrisch bei 570 nm bestimmt und die Probe Standard mit bekannten Konzentrationen mit der zu untersuchenden Probe verglichen und ausgewertet (Siehe Figuren 1 und 2).

**Tabelle 1**

| Verlauf der Säulentemperatur | | | | |
|---|---|---|---|---|
| Stufe | von | bis | Temperatur 1 | Temperatur 2 |
| | [h:min:sec] | [h:min:sec] | [°C] | [°C] |
| 1. | 00:00:00 | 00:12:40 | 33 | 33 |
| 2. | 00:12:40 | 00:36:36 | 33 | 33 |
| 3. | 00:36:36 | 00:47:02 | 33 | 44 |
| 4. | 00:47:02 | 01:11:15 | 44 | 33 |
| 5. | 01:11:15 | 01:43:57 | 33 | 59 |
| 6. | 01:43:57 | 02:06:34 | 59 | 40 |
| 7. | 02:06:34 | 02:58:15 | 40 | 66 |
| 8. | 02:58:15 | 03:04:56 | 66 | 70 |
| 9. | 03:04:56 | 03:13:36 | 70 | 70 |
| 10. | 03:13:36 | 03:21:22 | 70 | 35 |

### Statistische Auswertung

Die Auswertung erfolgte mit Hilfe des Programmsystems R-Studio Version 0.99.878. Zuerst erfolgte eine Überprüfung mit dem F-Test nach Fisher, ob die Zufallsvariablen (=Messreihen) identische Verteilung besitzen.

Der Shapiro-Wilk Test untersuchte anschließend, ob eine Zahlenreihe normalverteilt ist.

Unterschiede zwischen zwei Gruppen wurden mit dem T-Test bei Normalverteilung bzw. Wilcoxon-Test bei Nicht-Normalverteilung auf statistische Signifikanz getestet.

Das Signifikanzniveau wurde in Abhängigkeit von der Irrtumswahrscheinlichkeit α = 0,05 entsprechend auf p ≤ 0,05 festgelegt.

### Ergebnisdarstellung

In Tabelle 2 ist zu erkennen, dass ein Zusatz von Blutplasma in Schweinefleisch zu einer signifikanten Veränderung der freien Aminosäuren Ornithin und Cystin führt. Die anschließenden Mittelwertvergleiche zeigen, dass die Aminosäuren Ornithin und Cystin im Schweinefleisch (ohne Blutplasmazusatz) im Vergleich zu Schweinefleisch (mit 5% und 10% Blutplasmazusatz) eine signifikant erhöhte Konzentration aufweisen.

Weitere analytische Untersuchungen bei Brühwurst zeigten gleichfalls eine signifikante Erhöhung der Konzentrationen von den Aminosäuren Ornithin und Cystin bei Zusatz von 5% und 10% Blutplasma (Tabelle 3). In der Tabelle 4 ist zu erkennen, dass der Ornithin / Cystin Quotient von Schweinefleisch im Vergleich zu Schweinefleisch mit Blutplasmazusatz sich nicht verändert und in der gleichen Größenordnung liegt. Diese Tatsache spiegelt sich auch bei Brühwurst und Brühwurst mit Blutplasmazusatz wieder (Tabelle 5).

Die beiden Aminosäuren Ornithin (Orn) und Cystin (Cys)2 sind relevant für eine aussagefähige Beurteilung

**Tabelle: 2**

| Konzentrationen in freiem Cystin und Ornithin in nmol / g Schweinefleisch pur und mit Blutplasmazusatz | | | | | | |
|---|---|---|---|---|---|---|
| AA | Schweinefleisch pur | | Schweinefleisch plus 5% Plasma | | Schweinefleisch plus 10% Plasma | |
| | Mittelwert | SD | Mittelwert | SD | Mittelwert | SD |
| Cystin | 40,47ab | ± 2,28 | 46, 1a | ± 2,37 | 51,83b | ± 3,39 |
| Ornithin | 73,12ab | ± 4,76 | 84,87a | ± 5,28 | 95,74b | ± 6,01 |

Werte sind Mittelwerte ± Standardabweichung (n = 20)
a, b Mittelwerte mit unterschiedlichen Hochbuchstaben unterscheiden sich signifikant voneinander p ≤ 0,05

**Tabelle: 3**

| Konzentrationen in freiem Cystin und Ornithin in nmol / g Brühwurst pur und mit Blutplasmazusatz | | | | | | |
|---|---|---|---|---|---|---|
| AA | Brühwurst pur | | Brühwurst plus 5% Plasma | | Brühwurst plus 10% Plasma | |
| | Mittelwert | SD | Mittelwert | SD | Mittelwert | SD |
| Cystin | 37,54ab | ± 3,35 | 43,42a | ± 4,26 | 49,07b | ± 3,69 |
| Ornithin | 65,16 ab | ± 4,26 | 74,32a | ± 4,16 | 85,49b | ± 5,16 |

Werte sind Mittelwerte ± Standardabweichung (n = 20)
a,b Mittelwerte mit unterschiedlichen Hochbuchstaben unterscheiden sich signifikant voneinander p ≤ 0,05

**Tabelle: 4**

| Quotient Ornithin / Cystin von Schweinefleisch pur und mit Blutplasmazusatz | | | | | | |
|---|---|---|---|---|---|---|
| Quotient | Schweinefleisch pur | | Schweinefleisch plus 5% Plasma | | Schweinefleisch plus 10% Plasma | |
| | Mittelwert | SD | Mittelwert | SD | Mittelwert | SD |
| OM/Cys | 1,81 | ± 0,027 | 1,84 | ± 0,028 | 1,85 | ± 0,036 |

**Tabelle: 5**

| Quotient Ornithin / Cystin von Brühwurst pur und mit Blutplasmazusatz | | | | | | |
|---|---|---|---|---|---|---|
| Quotient | Brühwurst pur | | Brühwurst plus 5% Plasma | | Brühwurst plus 10% Plasma | |
| | Mittelwert | SD | Mittelwert | SD | Mittelwert | SD |
| Om/Cys | 1,73 | ± 0,048 | 1,71 | ± 0,074 | 1,74 | ± 0,034 |

Die in den Tabellen 1 bis 5 aufgeführten Werte zeigen auf beeindruckende Weise, dass das erfindungsgemäße Verfahren signifikante Ergebnisse liefert.

Bei einem Zusatz von Blutplasma in Fleisch und Fleischerzeugnisse steigen die Konzentrationen von den freien Aminosäuren Ornithin und Cystin im Verhältnis zur zugegebenen Blutplasmamenge, mithin wird der Orn/Cys-Quotient nicht verändert. Daher sind die Aminosäuren Ornithin und Cystin geeignete Marker, um einen Zusatz von Blutplasma in Fleischerzeugnissen nachzuweisen.

## Patentansprüche

1. Verfahren zum qualitativen Nachweis von zugesetztem Blutplasma in Lebensmitteln in Fleisch und Fleischwaren, wobei die Lebensmittel bei der Probenaufbereitung in Präzipitat- und Fluid-Phasen getrennt werden, wobei zumindest ein Teil der Fluidphase auf Aminosäuren qualitativ und quantitativ untersucht wird, wobei die ermittelten Probengehalte dieser Fluidphasen-Aminosäuren verglichen werden mit solchen Probengehalten, die aus den entsprechenden, nicht mit Blutplasma behandelten Lebensmitteln stammen, wobei bei der Untersuchung auf zugesetztes Blutplasma in der Fluidphase die Aminosäuren Ornithin und Cystin berücksichtigt werden,
**dadurch gekennzeichnet, dass**
es sich beim Verfahren um ein, mehrere Eluenten und ein Trennprogramm verwendendes chromatographisches Verfahren handelt, wobei es sich bei den Eluenten um folgende handelt, mit jeweils folgender relativer Zusammensetzung:
A: Lithiumacetat-Dihydrat 6,65 g, Ethanol 10 ml, Trifluoressigsäure-Lsg 50 % 8 ml, Caprylsäure 0,1 ml, Reinstwasser 470 ml.
B: Lithiumhydroxid-Monohydrat 2,5 g, Borsäure 0,5 g , Ethanol 5 ml, Caprylsäure 0,1 ml, Trifluoressigsäure- Lsg. 50% 4,7 ml, Reinstwasser 480 ml.
C: Lithiumhydroxid-Monohydrat 2,1 g, Borsäure 1,5 g Ameisensäure 1,5 ml, Essigsäure 100% 1,5 ml, Caprylsäure 0,1 ml, Trifluoressigsäure-Lsg. 50% 0,8 ml, Reinstwasser 495 ml.
D: Di-Lithiumtetraborat 2,0 g, Borsäure 2,5 g, EthylendiaminTetraessigsäure Di-Lithiumsalz Hydrat 0,13 g, Lithiumhydroxid-Monohydrat 3,18 g, Caprylsäure 0,1 ml, Trifluoressigsäure 50% 3 ml, Reinstwasser 483 ml.
E: Di-Lithiumtetraborat 1,0 g, Borsäure 1,5 g, LithiumhydroxidMonohydrat 5,25 g, Caprylsäure 0,1 ml, Trifluoressigsäure 50% 3,5 ml, Reinstwasser 490 ml.

2. Verfahren nach Anspruch 1, **dadurch gekennzeichnet, dass** die aus zumindest einem Teil der Fluidphase stammenden freien Aminosäuren qualitativ und quantitativ bestimmt werden, wobei die ermittelten Probengehalte dieser freien Aminosäuren verglichen werden mit solchen Probengehalten, die aus den entsprechenden, nicht mit Blutplasma behandelten Lebensmitteln stammen.

3. Verfahren nach einem der Ansprüche 1 bis 2, **dadurch gekennzeichnet, dass** für einen Zusatz von Blutplasma Probengehalte freier Aminosäuren, die sich durch den Zusatz erhöhen, mit Probengehalten freier Aminosäuren, die durch den Zusatz praktisch unverändert bleiben, zur Durchführung des Vergleichs in Beziehung gesetzt werden.

4. Verfahren nach einem der Ansprüche 1 bis 3, **dadurch gekennzeichnet, dass** bei der Probenaufbereitung die in den Lebensmitteln enthaltenen freien Aminosäuren von den in den Lebensmitteln enthaltenen Proteinen getrennt werden.

5. Verfahren nach einem der Ansprüche 1 bis 4, **dadurch gekennzeichnet, dass** die in den Lebensmitteln enthaltenen Proteine vor Trennung von den freien Aminosäuren zunächst aus den Lebensmitteln ausgefällt werden.

6. Verfahren nach einem der Ansprüche 1 bis 5, **dadurch gekennzeichnet, dass** die ausgefällten Proteine vor der Bestimmung der freien Aminosäuren von diesen abfiltriert werden.

7. Verfahren nach einem der Ansprüche 1 bis 6, **dadurch gekennzeichnet, dass** vor dem Filtrieren die Proteine und die freien Aminosäuren einer Zentrifugierung unterworfen werden.

8. Verfahren nach einem der Ansprüche 1 bis 7, **dadurch gekennzeichnet, dass** die Lebensmittel mittels Zugabe von Sulfosalicylsäure in Präzipitat- und Fluid-Phasen getrennt werden.

9. Verfahren nach einem der Ansprüche 1 bis 8, **dadurch gekennzeichnet, dass** der nahezu gleichbleibende Quotient der Molmengen von Ornithin zu Cystin oder von Cystin zu Ornithin als Indikator, dass Blutplasma zugesetzt worden ist, herangezogen wird.

10. Verfahren nach Anspruch 9, **dadurch gekennzeichnet, dass** es sich bei dem zu untersuchenden Lebensmittel um Schweinefleisch und/oder um Schweinefleischwaren handelt.

11. Verfahren nach einem der Ansprüche 1 bis 10, **dadurch gekennzeichnet, dass** die Eluenten bei den nachfolgenden pH-Werten verwendet werden: Eluent A pH 2,96, Eluent B pH 3,46, Eluent C pH 4,48, Eluent D pH 7,76, Eluent E pH 10,42.

12. Verfahren nach einem der Ansprüche 1 bis 11, **dadurch gekennzeichnet, dass** die Eluenten A bis E nacheinander in der Reihenfolge A, B, C, D, E verwendet werden.

13. Verfahren nach einem der Ansprüche 1 bis 12, **dadurch gekennzeichnet, dass** das folgende Trennprogramm verwendet wird, wobei die Daten sich auf eine Eluentenflussrate von 220 µl/min und folgenden Eluenten- und Säulentemperaturverlauf beziehen:
- Gesamtlaufzeit: 205 Minuten, 20 Sekunden;
Eluentenverlauf:
- Eluent A Zeit ab 0 Stunden, 0 Minuten bis 36 Minuten, 35 Minuten
- Eluent B Zeit ab 36 Minuten, 35 Sekunden bis 1 Stunde, 2 Minuten, 42 Sekunden
- Eluent C Zeit ab 1 Stunde, 2 Minute, 42 Sekunden bis 1 Stunde, 32 Minuten, 21 Sekunden
- Eluent D Zeit ab 1 Stunde, 32 Minuten, 21 Sekunden bis 2 Stunden, 2 Minuten, 9 Sekunden
- Eluent E Zeit ab 2 Stunden, 2 Minuten, 9 Sekunden bis 2 Stunden, 51 Minuten, 36 Sekunden
- Säulentemperaturverlauf:
- Zeit: 0 Stunden, 0 Minuten bis 12 Minuten, 40 Sekunden; Säulentemperatur: + 33°C bei 0 Stunden, 0 Minuten; + 33°C bei 12 Minuten, 40 Sekunden;
- Zeit: 12 Minuten, 40 Sekunden bis 36 Minuten, 36 Sekunden; Säulentemperatur: + 33°C bei 12 Minuten, 40 Sekunden; + 33°C bei 36 Minuten, 36 Sekunden;
- Zeit: 36 Minuten, 36 Sekunden bis 47 Minuten, 02 Sekunden; Säulentemperatur: + 33°C bei 36 Minuten, 36 Sekunden; + 44°C bei 47 Minuten, 02 Sekunden;
- Zeit: 47 Minuten, 02 Sekunden bis 1 Stunden, 11 Minuten, 15 Sekunden; Säulentemperatur: + 44°C bei 47 Minuten, 02 Sekunden; + 33°C bei 1 Stunde, 11 Minuten, 15 Sekunden;
- Zeit: 1 Stunde, 11 Minuten, 15 Sekunden bis 1 Stunde, 43 Minuten, 57 Sekunden; Säulentemperatur: + 33°C bei 1 Stunde, 11 Minuten, 15 Sekunden; + 59°C bei 1 Stunde, 43 Minuten, 57 Sekunden;
- Zeit: 1 Stunde, 43 Minuten, 57 Sekunden bis 2 Stunden, 06 Minuten, 34 Sekunden; Säulentemperatur: + 59°C bei 1 Stunde, 43 Minuten, 57 Sekunden; + 40 °C bei 2 Stunden, 06 Minuten, 34 Sekunden;
- Zeit: 2 Stunden, 06 Minuten, 34 Sekunden bis 2 Stunden, 58 Minuten, 15 Sekunden; Säulentemperatur: + 40 °C bei 2 Stunden, 58 Minuten, 15 Sekunden; + 66 °C bei 2 Stunden, 58 Minuten, 15 Sekunden;
- Zeit: 2 Stunden, 58 Minuten, 15 Sekunden bis 3 Stunden, 04 Minuten, 56 Sekunden; Säulentemperatur: + 66°C bei 2 Stunden, 58 Minuten, 15 Sekunden; + 70°C bei 3 Stunden, 04 Minuten, 56 Sekunden;
- Zeit: 3 Stunden, 04 Minuten, 56 Sekunden bis 3 Stunden, 13 Minuten, 36 Sekunden; Säulentemperatur: + 70°C bei 3 Stunden, 04 Minuten, 56 Sekunden; + 70°C bei 3 Stunden, 13 Minuten, 36 Sekunden;
- Zeit: 3 Stunden, 13 Minuten, 36 Sekunden bis 3 Stunden, 21 Minuten, 22 Sekunden; Säulentemperatur: + 70°C bei 3 Stunden, 13 Minuten, 36 Sekunden; + 35°C bei 3 Stunden, 21 Minuten, 22 Sekunden.

14. Verwendung von Ornithin und Cystin als Indikator zum qualitativen Nachweis von zugesetztem Blutplasma in Lebensmitteln in Fleisch und Fleischwaren, in einem Verfahren nach einem der Ansprüche 1 bis 13.

15. Verwendung nach Anspruch 14, **dadurch gekennzeichnet, dass** auch der nahezu konstante Quotient der Molmengen von Ornithin zu Cystin oder umgekehrt berücksichtigt wird.

16. Verwendung nach einem der Ansprüche 14 bis 15, **dadurch gekennzeichnet, dass** es sich bei den zu untersuchenden Lebensmitteln um Schweinefleisch und/oder um Schweinefleischwaren handelt.

17. Verwendung eines Eluenten, aufgezeigt in Anspruch 1, in einem Verfahren nach einem der Ansprüche 1 bis 13.

## Claims

1. Method for qualitative detection of added blood plasma in foodstuffs in meat and meat products, wherein during sample preparation, the foodstuffs are separated into precipitate phases and fluid phases, wherein at least some of the fluid phase is investigated qualitatively and quantitatively for amino acids, wherein the ascertained sample contents of these fluid phase amino acids are compared with such sample contents which come from the corresponding foodstuffs not treated with blood plasma, wherein in the investigation for added blood plasma in the fluid phase, the amino acids ornithine and cysteine are taken into account,
- **characterised in that** the method is a chromatographic method using several eluents and a separating programme, wherein the eluents are the following with respectively the following relevant composition:
- A: Lithium acetate dihydrate 6.65 g, ethanol 10 ml, trifluoroacetic acid solution 50% 8 ml. caprylic acid 0.1 ml, ultrapure water 470 ml.
- B: Lithium hydroxide monohydrate 2.5 g, boric acid 0.5 g, ethanol 5 ml, caprylic acid 0.1 ml, trifluoroacetic acid solution 50% 4.7 ml, ultrapure water 480 ml.
- C: Lithium hydroxide monohydrate 2.1 g, boric acid 1.5 g, formic acid 1.5 ml, acetic acid 100% 1.5 ml, caprylic acid 0.1 ml, trifluoroacetic acid solution 50% 0.8 ml, ultrapure water 495 ml.
- D: Di-lithium tetraborate 2.0 g, boric acid 2.5 g, ethylenediaminetetraacetic acid di-lithium salt hydrate 0.13 g, lithium hydroxide monohydrate 3.18 g, caprylic acid 0.1 ml, trifluoroacetic acid 50% 3 ml, ultrapure water 483 ml.
- E: Di-lithium tetraborate 1.0 g, boric acid 1.5 g, lithium hydroxide monohydrate 5.25 g, caprylic acid 0.1 ml, trifluoroacetic acid 50% 3.5 ml, ultrapure water 490 ml.

2. Method according to claim 1, **characterised in that** the free amino acids coming from at least some of the fluid phase are determined qualitatively and quantitatively, wherein the ascertained sample contents of these free amino acids are compared with such sample contents which come from the corresponding foodstuffs not treated with blood plasma.

3. Method according to one of claims 1 to 2, **characterised in that** for an addition of blood plasma, sample contents of free amino acids, which are increased by the addition, are related to sample contents of free amino acids, which remain practically unchanged by the addition, to carry out the comparison.

4. Method according to one of claims 1 to 3, **characterised in that** during sample preparation, the free amino acids present in the foodstuffs are separated from the proteins present in the foodstuffs.

5. Method according to one of claims 1 to 4, **characterised in that** the proteins present in the foodstuffs are precipitated first of all from the foodstuffs before separation from the free amino acids.

6. Method according to one of claims 1 to 5, **characterised in that** before determination of the free amino acids, the precipitated proteins are filtered off from the latter.

7. Method according to one of claims 1 to 6, **characterised in that** before filtering, the proteins and the free amino acids are subjected to centrifuging.

8. Method according to one of claims 1 to 7, **characterised in that** the foodstuffs are separated into precipitate phases and fluid phases by means of addition of sulphosalicylic acid.

9. Method according to one of claims 1 to 8, **characterised in that** the almost constant quotient of the molar quantities of ornithine to cysteine or of cysteine to ornithine is used as an indicator that blood plasma has been added.

10. Method according to claim 9, **characterised in that** the foodstuff to be investigated is pork and/or pork products.

11. Method according to one of claims 1 to 10, **characterised in that** the eluents are used at the following pH values: eluent A pH 2.96, eluent B pH 3.46, eluent C pH 4.48, eluent D pH 7.76, eluent E pH 10.42.

12. Method according to one of claims 1 to 11, **characterised in that** the eluents A to E are used one after another in the sequence A, B, C, D, E.

13. Method according to one of claims 1 to 12, **characterised in that** the following separating programme is used, wherein the data relate to an eluent flow rate of 220 µl/minute and the following eluent profile and column temperature profile:
∘ total running time: 205 minutes, 20 seconds;
- eluent profile:
∘ eluent A time from 0 hours, 0 minutes to 36 minutes, 35 minutes
∘ eluent B time from 36 minutes, 35 seconds to 1 hour, 2 minutes, 42 seconds
∘ eluent C time from 1 hour, 2 minutes, 42 seconds to 1 hour, 32 minutes, 21 seconds
∘ eluent D time from 1 hour, 32 minutes, 21 seconds to 2 hours, 2 minutes, 9 seconds
∘ eluent E time from 2 hours, 2 minutes, 9 seconds to 2 hours, 51 minutes, 36 seconds
- column temperature profile:
∘ time: 0 hours, 0 minutes to 12 minutes, 40 seconds; column temperature: + 33°C at 0 hours, 0 minutes; + 33°C at 12 minutes, 40 seconds;
∘ time: 12 minutes, 40 seconds to 36 minutes, 36 seconds; column temperature: + 33°C at 12 minutes, 40 seconds; + 33°C at 36 minutes, 36 seconds;
∘ time: 36 minutes, 36 seconds to 47 minutes, 02 seconds; column temperature: + 33°C at 36 minutes, 36 seconds; + 44°C at 47 minutes, 02 seconds;
∘ time: 47 minutes, 02 seconds to 1 hour, 11 minutes, 15 seconds; column temperature: + 44°C at 47 minutes, 02 seconds; + 33°C at 1 hour, 11 minutes, 15 seconds;
∘ time: 1 hour, 11 minutes, 15 seconds to 1 hour, 43 minutes, 57 seconds; column temperature: + 33°C at 1 hour, 11 minutes, 15 seconds; + 59°C at 1 hour, 43 minutes, 57 seconds;
∘ time: 1 hour, 43 minutes, 57 seconds to 2 hours, 06 minutes, 34 seconds; column temperature: + 59°C at 1 hour, 43 minutes, 57 seconds; + 40°C at 2 hours, 06 minutes, 34 seconds;
∘ time: 2 hours, 06 minutes, 34 seconds to 2 hours, 58 minutes, 15 seconds; column temperature: + 40°C at 2 hours, 58 minutes, 15 seconds; + 66°C at 2 hours, 58 minutes, 15 seconds;
∘ time: 2 hours, 58 minutes, 15 seconds to 3 hours, 04 minutes, 56 seconds; column temperature: + 66°C at 2 hours, 58 minutes, 15 seconds; + 70°C at 3 hours, 04 minutes, 56 seconds;
∘ time: 3 hours, 04 minutes, 56 seconds to 3 hours, 13 minutes, 36 seconds; column temperature: + 70°C at 3 hours, 04 minutes, 56 seconds; + 70°C at 3 hours, 13 minutes, 36 seconds;
∘ time: 3 hours, 13 minutes, 36 seconds to 3 hours, 21 minutes, 22 seconds; column temperature: + 70°C at 3 hours, 13 minutes, 36 seconds; + 35°C at 3 hours, 21 minutes, 22 seconds.

14. Use of ornithine and cysteine as an indicator for qualitative detection of added blood plasma in foodstuffs in meat and meat products in a method according to one of claims 1 to 13.

15. Use according to claim 14, **characterised in that** also the almost constant quotient of the molar quantities of ornithine to cysteine or vice versa is taken into account.

16. Use according to one of claims 14 to 15, **characterised in that** the foodstuffs to be investigated are pork and/or pork products.

17. Use of an eluent shown in claim 1 in a method according to one of claims 1 to 13.

## Revendications

1. Procédé pour la détection qualitative du plasma sanguin ajouté dans des produits alimentaires, viandes et produits de viande, les produits alimentaires étant, lors de la préparation des échantillons, séparés en les phases précipité et fluide, au moins une partie de la phase fluide faisant l'objet d'un dosage qualitatif et quantitatif des acides aminés, les teneurs déterminées des échantillons en ces acides aminés de la phase fluide étant comparées aux teneurs des échantillons qui proviennent des produits alimentaires correspondants, non traités par du plasma sanguin, les acides aminés dans la phase fluide, ornithine et cystine, étant pris en considération lors de examen du plasma sanguin ajouté,
- **caractérisé en ce que**, pour ce qui concerne le procédé, il s'agit d'un procédé chromatographique utilisant plusieurs éluants et un programme de séparation, les éluants étant les suivants, ayant chacun la composition relative suivante:
- A: acétate de lithium dihydraté 6,65 g, éthanol 10 ml, solution d'acide trifluoracétique à 50 % 8 ml, acide caprylique 0,1 ml, eau ultrapure 470 ml.
- B: hydroxyde de lithium monohydraté 2,5 g, acide borique 0,5 g, éthanol 5 ml, acide caprylique 0,1 ml, solution d'acide trifluoracétique à 50 % 4,7 ml, eau ultrapure 480 ml.
- C: hydroxyde de lithium monohydraté 2,1 g, acide borique 1,5 g, acide formique 1,5 ml, acide acétique à 100 % 1,5 ml, acide caprylique 0,1 ml, solution d'acide trifluoracétique à 50 % 0,8 ml, eau ultrapure 495 ml.
- D: tétraborate de dilithium 2,0 g, acide borique 2,5 g, sel de dilithium hydraté de l'acide éthylènediaminetétraacétique 0,13 g, hydroxyde de lithium monohydraté 3,18 g, acide caprylique 0,1 ml, acide trifluoracétique à 50 % 3 ml, eau ultrapure 483 ml.
- E: tétraborate de dilithium 1,0 g, acide borique 1,5 g, hydroxyde de lithium monohydraté 5,25 g, acide caprylique 0,1 ml, acide trifluoracétique à 50 % 3,5 ml, eau ultrapure 490 ml.

2. Procédé selon la revendication 1, **caractérisé en ce que** les acides aminés libres provenant d'au moins une partie de la phase fluide font l'objet d'une détermination qualitative et quantitative, les teneurs déterminées des échantillons en ces acides aminés libres étant comparées aux teneurs des échantillons qui proviennent des produits alimentaires correspondants, non traités par du plasma sanguin.

3. Procédé selon l'une des revendications 1 à 2, **caractérisé en ce que**, pour une addition de plasma sanguin, on met en relation, pour la mise en œuvre de la comparaison, les teneurs des échantillons en acides aminés libres qui augmentent sous l'effet de l'addition, avec les teneurs des échantillons en acides aminés libres qui restent pratiquement inchangées sous l'effet de l'addition.

4. Procédé selon l'une des revendications 1 à 3, **caractérisé en ce que**, lors de la préparation des échantillons, on sépare les acides aminés libres contenus dans les produits alimentaires d'avec les protéines contenues dans les produits alimentaires.

5. Procédé selon l'une des revendications 1 à 4, **caractérisé en ce que** les protéines contenues dans les produits alimentaires sont d'abord, avant séparation d'avec les acides aminés libres, séparées des produits alimentaires par précipitation.

6. Procédé selon l'une des revendications 1 à 5, **caractérisé en ce que** les protéines qui ont précipité sont, avant détermination des acides aminés libres, séparées de ces derniers par filtration.

7. Procédé selon l'une des revendications 1 à 6, **caractérisé en ce que**, avant la filtration, les protéines et les acides aminés libres sont soumis à une centrifugation.

8. Procédé selon l'une des revendications 1 à 7, **caractérisé en ce que** les produits alimentaires sont séparés en des phases précipité et fluide par addition d'acide sulfosalicylique.

9. Procédé selon l'une des revendications 1 à 8, **caractérisé en ce qu'**on utilise le rapport presque constant entre les quantités en moles de l'ornithine et de la cystine ou de la cystine et de l'ornithine en tant qu'indicateur de ce que l'on a ajouté du plasma sanguin.

10. Procédé selon la revendication 9, **caractérisé en ce que**, pour ce qui concerne le produit alimentaire à examiner, il s'agit de viande de porc et/ou de produits de viande de porc.

11. Procédé selon l'une des revendications 1 à 10, **caractérisé en ce que** les éluants sont utilisés aux pH suivants: éluant A pH 2,96, éluant B pH 3,46, éluant C pH 4,48, éluant D pH 7,76, éluant E pH 10,42.

12. Procédé selon l'une des revendications 1 à 11, **caractérisé en ce que** les éluants A à E sont utilisés les uns après les autres dans l'ordre A, B, C, D, E.

13. Procédé selon l'une des revendications 1 à 12, **caractérisé en ce qu'**on utilise le programme de séparation suivant, les données se rapportant à un débit d'éluant de 220 µl/min, et au programme suivant de températures des éluants et de la colonne
∘ durée totale d'élution: 205 minutes, 20 secondes;
- programme des éluants:
∘ temps de l'éluant A de 0 heure, 0 minute à 36 minutes, 35 minutes
∘ temps de l'éluant B de 36 minutes, 35 secondes à 1 heure, 2 minutes, 42 secondes
∘ temps de l'éluant C de 1 heure, 2 minutes, 42 secondes à 1 heure, 32 minutes, 21 secondes
∘ temps de l'éluant D de 1 heure, 32 minutes, 21 secondes à 2 heures, 2 minutes, 9 secondes
∘ temps de l'éluant E de 2 heures, 2 minutes, 9 secondes à 2 heures, 51 minutes, 36 secondes
- programme des températures de colonne
∘ temps: 0 heure, 0 minute à 12 minutes, 40 secondes; température de colonne: + 33 °C à 0 heure, 0 minute; + 33 °C à 12 minutes, 40 secondes;
∘ temps: 12 minutes, 40 secondes à 36 minutes, 36 secondes; température de colonne: + 33 °C à 12 minutes, 40 secondes; + 33 °C à 36 minutes, 36 secondes;
∘ temps: 36 minutes, 36 secondes à 47 minutes, 02 seconde; température de colonne: + 33 °C à 36 minutes, 36 secondes; 44 °C à 47 minutes, 0,2 seconde;
∘ temps: 47 minutes, 0,2 seconde à 1 heure, 11 minutes, 15 secondes; température de colonne: + 44 °C à 47 minutes, 0,2 seconde; + 33 °C à 1 heure, 11 minutes, 15 secondes;
∘ temps: 1 heure, 11 minutes, 15 secondes à 1 heure, 43 minutes, 57 secondes; température de colonne: + 33 °C à 1 heure, 11 minutes, 15 secondes; + 59 °C à 1 heure, 43 minutes, 57 secondes;
∘ temps: 1 heure, 43 minutes, 57 secondes à 2 heures, 0,6 minute, 34 secondes; température de colonne: + 59 °C à 1 heure, 43 minutes, 57 secondes; + 40 °C à 2 heures, 0,6 minute, 34 secondes;
∘ temps: 2 heures, 0,6 minute, 34 secondes à 2 heures, 58 minutes, 15 secondes; température de colonne: + 40 °C à 2 heures, 58 minutes, 15 secondes; + 66 °C à 2 heures, 58 minutes, 15 secondes;
∘ temps: 2 heures, 58 minutes, 15 secondes à 3 heures, 0,4 minute, 56 secondes; température de colonne: + 66 °C à 2 heures, 58 minutes, 15 secondes; + 70 °C à 3 heures, 0,4 minute, 56 secondes;
∘ temps: 3 heures, 0,4 minute, 56 secondes à 3 heures, 13 minutes, 36 secondes; température de colonne: + 70 °C à 3 heures, 0,4 minute, 56 secondes; + 70 °C à 3 heures, 13 minutes, 36 secondes;
∘ temps: 3 heures, 13 minutes, 36 secondes à 3 heures, 21 minutes, 22 secondes; température de colonne: + 70 °C à 3 heures, 13 minutes, 36 secondes; + 35 °C à 3 heures, 31 minutes, 22 secondes.

14. Utilisation d'ornithine et de cystine en tant qu'indicateurs pour la détection qualitative du plasma sanguin ajouté dans les produits alimentaires viande et produits de viande, dans un procédé selon l'une des revendications 1 à 13.

15. Utilisation selon la revendication 14, **caractérisée en ce qu'**on prend aussi en compte le rapport presque constant entre les quantités molaires de l'ornithine et de la cystine, ou inversement.

16. Utilisation selon l'une des revendications 14 à 15, **caractérisée en ce que**, pour ce qui concerne les produits alimentaires à examiner, il s'agit de viande de porc et/ou de produits de viande de porc.

17. Utilisation d'un éluant tel que présenté dans la revendication 1, dans un procédé selon l'une des revendications 1 à 13.
